**Europäisches Patentamt**

**European Patent Office**

**Office européen des brevets**

(11) Numéro de publication: **0 009 661**
**B1**

(12) **FASCICULE DE BREVET EUROPEEN**

(45) Date de publication du fascicule du brevet: **10.10.84**

(51) Int. Cl.³: **A 23 L 3/34, A 23 L 1/22**

(21) Numéro de dépôt: **79103340.0**

(22) Date de dépôt: **07.09.79**

(54) **Procédé de préparation de substances antioxygènes et leur utilisation.**

(30) Priorité: **29.09.78 CH 10186/78**

(43) Date de publication de la demande:
**16.04.80 Bulletin 80/08**

(45) Mention de la délivrance du brevet:
**10.10.84 Bulletin 84/41**

(84) Etats contractants désignés:
**AT DE FR IT NL**

(56) Documents cités:
**DE-A-2 445 354**
**US-A-3 732 111**
**US-A-3 908 031**

(73) Titulaire: **SOCIETE DES PRODUITS NESTLE S.A.**
**Case postale 353**
**CH-1800 Vevey (CH)**

(72) Inventeur: **Bracco, Umberto**
**Chemin de Béranges 18**
**CH-1814 La Tour-de-Peilz (CH)**
Inventeur: **Viret, Jean-Louis**
**Avenue de Jaman 19**
**CH-1814 La Tour-de-Peilz (CH)**
Inventeur: **Rehacek, Josef**
**Roselière 13**
**CH-1400 Yverdon (CH)**

(74) Mandataire: **Archambault, Jean**
**55, avenue Nestlé**
**CH-1800 Vevey (CH)**

Courier Press, Leamington Spa, England.

EP 0 009 661 B1

# 0 009 661

**Description**

La présente invention concerne un procédé de préparation d'une fraction contenant des substances antioxygènes à partir d'une matière végétale riche en ces substances et l'utilisation de la fraction ainsi obtenue pour préserver les produits alimentaires et cosmétiques de l'oxydation.

Les substances antioxygènes à usage alimentaire général ou cosmétique doivent avoir une bonne activité antioxygène, être stables, incolores, neutres organoleptiquement et leur inocuité doit être garantie.

On connait des substances antioxygènes obtenues par voie de synthèse dont l'usage est très répandu dans l'industrie alimentaire, généralement des substances phénoliques, par exemple le butylhydroxyanisol (B.H.A.) le butylhydroxytoluène (B.H.T.) seuls ou en mélange synergique. Bien que ces substances aient une activité antioxygène marquée, une bonne stabilité, qu'elles soient incolores et que leur inocuité et leur neutralité vis à vis des aliments soient reconnues, leur usage est fortement contesté par nombre de législations alimentaires.

On a également cherché à extraire les substances antioxygènes naturelles contenues dans les matières végétales.

Certains procédés proposent l'extraction directe des substances antioxygènes par voie chimique consistant en un traitement par une solution aqueuse basique.

Ces méthodes utilisant un agent chimique sont également actuellement contestées par les législations alimentaires. On leur préfère donc les procédés mettant en oeuvre une séparation purement physique des principes antioxygènes. Les extractions classiques de ces substances s'opèrent soit par des solvants organiques soit par des huiles végétales ou animales. Les méthodes par solvants impliquent la manipulation coûteuse de solvants dangereux souvent difficiles à éliminer et qui peuvent contaminer les substances antioxygènes obtenues. Les solvants ou les huiles présentent en outre l'inconvénient d'extraire également des substances colorantes, comme par exemple la clorophylle ou des principes aromatiques de la plante qu'il faut éliminer par des traitements supplémentaires de désodorisation et de décoloration. Ces traitements diminuent le rendement en principes antioxygènes et augmentent le coût de l'extraction.

Ainsi, un procédé récent décrit dans le brevet des Etats-Unis No. 3'732'111 concerne l'extraction des principes antioxygènes d'épices par broyage fin sur broyeur à meules, extraction à l'aide d'une huile végétale à température élevée, séparation par centrifugation et désodorisation par strippage à la vapeur sous vide à température élevée, les principes antioxygènes se retrouvant dans l'huile.

Une autre méthode décrite dans le brevet des Etats-Unis No 3'950'266 a trait à l'extraction des principes antioxygènes d'épices par traitement d'une poudre d'épice dans un premier stade par solvant à bas point d'ébullition, filtration et séchage, éventuellement décoloration sur charbon actif, filtration et séchage.

Dans un deuxième stade, l'extraite sec est repris par une huile végétale et soumis soit à. un strippage sous vide poussé, soit à une distillation moléculaire et ensuite éventuellement purifié par chromatographie en phase liquide.

Nous avons trouvé qu'on peut préparer à partir de matières végétales une fraction contenant des substances antioxygènes naturelles à usage alimentaire et cosmétique général, et répondant aux exigences mentionnées ci-dessus, par un traitement ne faisant pas intervenir d'étapes supplémentaires de décoloration ou désodorisation ou d'élimination de solvants nécessitant la séparation de résidus et donc génératrices de baisse importante du rendement en principes antioxygènes.

Le procédé selon l'invention est caractérisé par le fait

— qu'on met en suspension la matière végétale préalablement moulue dans une huile,

— qu'on micronise le mélange de manière à obtenir des particules de dimension d'au plus 20 microns,

— qu'on procède à un traitement thermique du mélange micronisé à 200—250°C pendant 1 à 5 minutes ou à 100—200°C pendant 5 à 10 minutes et qu'on sépare le résidu carbonisé, avant ou après y avoir ajouté un vecteur de distillation tel qu'un mono-, di- ou triglycéride à chaîne moyenne et éventuellement l'avoir standardisé par adjonction d'huile, de sorte que celui-ci contienne 10 à 30% en poids de matière végétale et 1 à 20% en poids de vecteur de distillation, et

— qu'on soumet la suspension traitée thermiquement à une distillation moléculaire sous un vide d'au plus 0,67 Pa à une température de 190—280°C et avec un débit de matière de 0,5 à 7 kg/h, et qu'on recueille un condensat contenant les principes antioxygènes, le vecteur de distillation et une partie de l'huile mise en oeuvre.

Par matière végétale, on entend les matières connues pour leur pouvoir antioxygène telles que les épices sous forme de feuilles, fleurs, fruits, racines, rhizômes et en particulier les plantes de la famille des labiacés, romarin, sauge, origan ou marjolaine, thym etc... mais également des résidus végétaux tels que les cosses, gousses, pelures, par exemple les pelures de cacao.

Une matière première particulièrement avantageuse est constituée par les résidus de l'extraction des huiles essentielles de sauge, et en particulier de romarin, par strippage.

2

Ces sous-produits de l'industrie de la parfumerie sont ordinairement jetés ou utilisés par les parfumeurs comme combustibles dans les chaudières de distillation.

Selon un mode d'exécution de l'invention, la matière végétale est préalablement broyée pour assurer sa dispersion convenable dans l'huile jusqu'à une dimension des particules de 100 à 400 microns.

On disperse ensuite les particules moulues dans une huile à raison de 10—30% en poids de matière végétale. On peut utiliser une huile d'origine animale ou végétale, mais on préfère une huile végétale qui a l'avantage d'être liquide à température ambiante et relativement neutre du point de vue couleur et odeur.

Selon un mode préféré de mise en oeuvre du procédé, on ajoute 0,5 à 5% en poids d'eau à la suspension destinée à la micronisation.

L'addition d'une faible quantité d'eau avant la micronisation permet une dispersion extrêmement fine de cette eau dans le mélange obtenu. Cette eau répartie de façon homogène facilite la désodorisation et la décoloration lors de la distillation moléculaire car, étant éliminée avec la fraction de tête de distillation, elle a pour fonction d'entraîner les principes colorants et odorants résiduels.

La suspension est ensuite soumise à une mouture très fine ou micronisation. On a constaté que la mouture très fine, les particules ayant une dimension de 5—20 microns, améliore la libération des substances antioxygènes contenues dans les cellules végétales par désintégration des parois cellulaires.

Les substances libérées se retrouvent ainsi dans l'huile. Un avantage très important de cette mouture très fine est qu'elle permet de traiter directement la suspension micronisée dans l'appareil de distillation moléculaire sans filtration préalable.

Toute méthode de micronisation permettant d'obtenir une désintégration des cellules végétales peut être utilisée. On préfère utiliser un moulin à billes qui permet un broyage poussé des cellules végétales jusqu'à obtenir des particules de 5—20 microns après ou un plusieurs passages. On peut déterminer le degré de désintégration des particules végétales par un choix adéquat de la dimension des billes, de la durée et de l'intensité du traitement. Pour certaines matières végétales contenant passablement de fibres, il peut subsister quelques particules de dimensions de quelques dizaines à quelques centaines de microns après une micronisation. On peut en variante, plutôt que d'opérer plusieurs micronisations successives, soumettre la suspension à une microfiltration de façon que la dimension des particules soit celle recherchée de 5—20 microns.

Pour préparer le mélange destiné à la distillation, la substance végétale sous forme de suspension huileuse micronisée, est mélangée avec un vecteur de distillation au co-distillant tel qu'un mono-, di- ou triglycéride à chaine moyenne à raison de 1 à 20% en poids du mélange préparé pour la distillation.

On a constaté que l'adjonction de ces produits avait plusieurs avantages:

— Etant entraînés avec les substances antioxygènes, les vecteurs viennent se déposer sur les condenseurs et permettent l'écoulement de ces substances et la récupération en continu du disillat.
— Lorsque l'on utilise un monoglycéride à pouvoir tensioactif élevé, celui-ci favorise le transfert des principes antioxygènes du solvant léger vers l'huile et ensuite le mélange intime de la fraction distillée contenant les principes antioxygènes, facilitant ainsi la manipulation et le dosage de ceux-ci.
— Ils facilitent la séparation des substances antioxygènes de l'huile ayant servi de solvant car ils sont entraînés avec ces substances.

On ajoute ensuite l'huile au mélange de la substance végétale et du co-distillant en quantité telle qu'elle représente 60 à 80% en poids du mélange total.

Lorsque la matière première est un résidu de la distillation des huiles essentielles du romarin, on a constaté qu'il est avantageux de procéder à un traitement thermique de la suspension micronisée. On a en effet remarqué la carbonisation d'une fraction représentant environ 5% en poids de l'extrait sec dans les conditions de température élevée de la distillation.

Pendant la distillation moléculaire, cette fraction a tendance à se déposer sur les condenseurs et souille le condensat. On chauffe la solution ou suspension à environ 200—250°C pendant 1 à 5 min, par exemple dans un échangeur à surface raclée, et on sépare la partie carbonisée, par exemple par tamisage ou contrifugation. La suspension est prête pour la distillation.

En variant, on peut soumettre la suspension à un traitement thermique n'allant pas jusqu'à la carbonisation, par exemple à température de 100—200°C pendant 5—10 minutes, l'opération de tamisage ou centrifugation constituant alors une option.

La distillation moléculaire de la suspension additionnée du vecteur de distillation et éventuellement débarrassé de la fraction susceptible de se carboniser peut avoir lieu dans tout appareil convenable tel qu'un évaporateur à film tombant ou à disque tournant à effet centrifuge. L'échantillon est maintenu en cuve d'attente à 60—90°C pous introduit dans l'appareil.

Si on utilise p. ex. un évaporateur à film tombant, celui-ci comporte un premier étage dont la surface d'évaporation est maintenue à 110—180°C, la surface de condensation étant à 80—160°C et dans lequel règne un vide de 0,67 à 26,66 Pa. Le vide peut être produit par une pompe à palettes couplée à des trappes par exemple à azote liquide maintenues à —196°C. Dans ce premier étage

3

s'opère simultanément un dégazage, une décoloration et une désodorisation. Comme indiquè ci-dessus, l'eau qui a été ajoutée au mélange sert à entraîner les principes colorants et odorants résiduels et est séparée comme premier condensat. Dans un deuxième étage dans lequel règne un vide inférieur à 0,67 Pa s'effectue la séparation de la fraction contenant les composés antioxygènes sous forme de condensat, la surface d'évaporation étant maintenue à 190—280°C cependant que la surface de condensation est à 80—160°C. Le distillat est constitué essentiellement de l'huile qui a servi comme solvant. Celui-ci peut être recyclé en tête de ligne et servir à nouveau pour la préparation du mélange à distiller, pour la micronisation ou pour la standardisation du du mélange micronisé.

Le débit du produit soumis à la distillation moléculaire peut varier de 0,5 à 7 Kg/h selon les conditions opératoires.

On a constaté que le rendement de l'extraction des principes antixoygènes est bien supérieur selon l'invention à celui des procédés analogues, car les suspensions soumises à la distillation moléculaire contiennant 5 à 20% en poids de la matière végétale de départ contre 1 à 2% en poids, par exemple pour les solutions traitées selon le brevet des Etats-Unis No. 3'950'266.

De plus, l'association de la micronisation dans l'huile et de la distillation moléculaire de la suspension permet d'isoler les principes antioxygènes de matières végétales sand extraction préalable, par exemple dans le cas des pelures de cacao plus ou moins séchées. Quelle que soit la matière première la fraction obtenue contient des principes antioxygènes à pouvoir antioxydant égal ou supérieur aux antioxygènes synthétiques. D'autre part, l'inocuité de celle-ci et sa relative neutralité organoleptique sont remarquables. Le procédé selon l'invention permet une conservation prolongée (d'au moins un an à température ambiante) des principes antioxygènes en solution concentrée (par exemple 40% en poids) sans aucune diminution du pouvoir antioxydant ni altération organoleptique, ce qui n'est absolument pas le cas des antioxydants chimiques classiques du type B.H.A. ou B.H.T.

L'invention concerne également l'utilisation de la fraction contenant des substances antioxygènes obtenues par le présent procédé pour protéger contre l'oxydation un aliment ou un produit cosmétique. Son incorporation dans les produits peut être réalisée par tout moyen connu, par exemple en solution, suspension ou émulsion dans des solvants, gaz liquéfiés ou autres, c'est-à-dire à l'aide d'un vecteur.

Comme aliments dans lesquels elle peut être incorporée on peut citer les matières grasses telles que les huiles végétales ou les graisses animales, les émulsions du type mayonnaise, pâte à tartiner, bouillons cubes, les produits humides contenant des matières grasses liées tels que la viande, le poisson ou des produits séchés, par exemple lyophilisés, les céreales, farines lactées, poudre de lait entier reconstitué, les légumes secs et particulièrement les flocons de pomme de terre.

Les produits cosmétiques craignant l'oxydation dans lesqueles la fraction antioxygène obtenue selon l'invention est avantageusement incorporée sont sous la forme de dispersions aqueuses (lotions, telles, que lotions avant ou après rasage), émulsions fluides (laits corporels, laits à démaquiller), crèmes (crème blanche, crème solaire), pâte (masque) et.... Leur protection contre l'oxydation permet en particulier d'éviter les problèmes olfactifs dus au rancissement.

Des résultats satisfaisants sont obtenus par une incorporation de 0,05 à 1% du condensat soit 0,01 à 0,2% de substance active entraînable, une partie du condensat étant constituée par l'huile entraîné et le vecteur de distillation.

Selon un mode d'utilisation préféré, on incorpore un mélange broyé du condensat obtenu selon l'invention avec 5 à 40% calculés sur le poids de condensat d'acide citrique ou ascorbique ou leurs sels et esters. On a en effet constaté qu'on augmentait ainsi de 7 à 12 fois le pouvoir antioxydant tel que mesuré en temps d'induction par la méthode ASTELL.

Les exemples non limitatifs qui suivent illustrent la mise en oeuvre du procédé selon l'invention. Dans céux-ci, les pourcentages et quantités sont pondéraux sauf indication contraire.

Exemple 1

Des aiguilles de romarin strippées provenant de l'extraction des huiles essentielles sont soumises à une mouture à sec dans un broyeur Alpine à disques distants de 0,8 mm avec rotor tournant à 900 tours par minute, jusqu'à obtenir des particules de 100—400 microns. 17 parties de romarin moulu sont mélangées à 83 parties d'huile d'arachide et on ajoute 0,5—5% d'eau au mélange. La suspension est traitée dans un moulin à billes Dino à disques agitateurs avec séparateur distant de 0,2 mm et vitesse circonférentielle des disques de 10 m/s, la chambre de broyage étant remplie aux quatre cinquèmes avec des billes de 1 à 1,5 mm de diamètre. La micronisation est effectuée en deux passages avec un temps de résidence de 3,5 min. On obtient ainsi une suspension de viscosité 0,21 Pa.s mesurée à 25°C.

La suspension micronisée est soumise à un traitement thermique par passage dans un échangeur à surface raclée à 200°C pendant une minute puis passée dans un tamis à mailles de diamètre 0,15 mm pour séparer la partie carbonisée correspondant à environ 5% de l'extrait sec.

On standardise par adjonction d'une quantité égale d'huile d'arachide et on ajoute 7 parties d'un monoglycéride (par exemple Dimodan S de Grindsted) pour 93 parties de la suspension micronisée standardisée qu'on transfère dans une cuve d'attente tempérée à 70°C.

La distillation moléculaire de la suspension est effectuée dans un évaporateur Leybold à film

4

tombant de 2 étages, ayant une surface active de l'echangeur de chaleur de 0,1 m² et une vitesse de rotation de 25—600 tours/min. dans les conditions suivantes:

| | |
|---|---|
| débit | 0,5 à 1 Kg/h |
| température des trappes | —196°C |
| température de la surface chauffée au premier étage | 120—160°C |
| température du condenseur au 1er étage | 90°C—150°C |
| vide au 1er étage | 0,005—0,67 Pa |
| température de la surface chauffée au 2ème étage | 200—230°C |
| température du condenseur au 2ème étage | 90°C—150°C |
| vide au 2ème étage | <0,001—0,13 Pa |

On recueille le condensat du 2ème étage de couleur ambre, de goût et d'odeur très faible représentant 8—12% (calculé sans le co-distillant) des feuilles de romarin traitées.

Exemple 2

On mesure l'absorption d'oxygène selon la méthode Astell de différents condensats obtenus par micronisation ou extraction et distillation moléculaire dans les conditions de l'exemple 1. Cette méthode consiste à mesurer l'absorption d'oxygène des graisses et des huiles au cours du temps dans des conditions précises de température et d'enregistrer la période d'induction par rapport à un échantillon de référence. Elle est décrite dans "BFMIRA Tech. Circular No. 487, July 1971, Meora M. L., Rosie D. A., a sensitive oxygen absorption apparatus for study the stability of fats".

Le substrat est 4 g de graisse de poule contenant 1000 ppm d'extrait rapporté à la quantité de substance antioxygène entraînable contenue dans la matière végétale, l'atmosphère au dessus de l'échantillon étant de l'air tandis que la température est de 90°C. Les résultats obtenus sont consignés dans le tableau IV ci-dessous:

TABLEAU IV

| Nature de l'extrait | Période d'induction (heures) |
|---|---|
| Graisse de poule sans substance antioxygène | 3—4 |
| graisse de poule+1000 ppm de substance active entraînable du romarin selon l'exemple 1 | 18—20 |
| graisse de poule+1000 ppm de substance active en entraînable de la sauge obtenue selon l'exemple 1 | 25—30 |
| graisse de poule+1000 ppm de substance active en entraînable des pelures de cacao obtenue selon l'exemple 1 | 18—20 |
| graisse de poule+1000 ppm de mélange à poids égal de butylhydroxyanisol (BHA) et de butylhydroxytoluène (BHT) | 20—25 |

Exemple 3

Une quantité correspondant à 300 ppm de substance active entraînable du romarin obtenue comme décrit dans l'exemple 1 est ajoutée à des flocons de pommes de terre, au cours de la fabrication de la purée de pommes de terre instantanée.

L'évolution de la dégradation des lipides contenus dans ce produit est suivie par mesure de la formation de pentane dans l'espace-de-tête, qui provient de la dégradation de l'acide octadécadiènoique présent, le pentane étant déterminé par chromatographie en phase gazeuse selon la méthode décrite par M. Arnaud et J. J. Wuhrmann, "Dehydrated food oxidation as measured by thermal release of hydrocarbons", 4th International Congress of Food Science and technology, Madrid 23—27.9.1974.

L'efficacité du pouvoir antioxydant des condensats est démontrée dans le tableau V suivant

## TABLEAU V

### Flocons de pommes-de-terre

| Stockage mois à 30°C | sans addition | UI×10³ Pentane avec addition 300 ppm de substance active entrainable |
|---|---|---|
| 0 | 0 | 0 |
| 1 | 8 | 0,2 |
| 2 | 17 | 4 |
| 3 | 26 | 8,5 |

Exemple 4

Une quantité de condensat du romarin obtenu comme décrit dans l'exemple 1 correspondant à 500 ppm de substance active entraînable, est ajoutée à une farine de blé pré-cuite avant séchage final. L'évolution de la dégradation lipidique dans le produit est suivie comme à l'exemple précédent et l'efficacité de l'antioxydant est déterminée avec les résultats indiqués dans le tableau VI ci-dessous:

## TABLEAU VI
### Farine de blé pré-cuite

| mois stockage à 30°C | sans addition | UI×10⁵ Pentane avec addition 500 ppm |
|---|---|---|
| 0 | 0 | 0 |
| 1 | 3,6 | 1,8 |
| 2 | 8,9 | 2,1 |
| 3 | 13,6 | 4,7 |

**Revendications**

1. Procédé de préparation d'une fraction contenant des substances antioxygènes à partie d'une matière végétale riche en ces substances, caractérisé par le fait

— qu'on met en suspension la matière végétale préalablement moulue dans une huile,
— qu'on micronise le mélange de manière à obtenir des particules de dimension d'au plus 20 microns,
— qu'on procède à un traitement thermique du mélange micronisé à 200—250°C pendant 1 à 5 minutes ou à 100—200°C pendant 5 à 10 minutes et qu'on sépare le résidu carbonisé, avant ou après y avoir ajouté un vecteur de distillation tel qu'on mono-, di- ou triglycéride à chaîne moyenne et éventuellement l'avoir standardisé par adjonction d'huile, de sorte que celui-ci contienne 10 à 30% de matière végétale et 1 à 20% en poids de vecteur de distillation, et
— qu'on soumet la suspension traitée thermiquement à une distillation moléculaire sous un vide d'au plus 0,67 Pa à une température de 190—280°C et avec un débit de matière de 0,5 à 7 kg/h, et qu'on recueille un condensat contenant les principes antioxygènes, le vecteur de distillation et une partie de l'huile mise en oeuvre.

2. Procédé selon la revendication 1, caractérisé en ce que la matière végétale traitée est constituée de feuilles de romarin ou de sauge ou de pelures de cacao.

3. Procédé selon la revendication 1, caractérisé en ce que la matière végétale traitée est constituée de résidus de la distillation de l'huile essentielle de romarin.

4. Procédé selon la revendication 1, caractérisé en ce qu'on ajoute à la suspension 0,5 à 5% en poids d'eau avant micronisation.

5. Utilisation de la fraction contenant des substances antioxygènes obtenues par le procédé selon l'une des revendications 1 à 4 pour protéger contre l'oxydation un produit alimentaire ou cosmétique, caractérisée par le fait qu'on incorpore de 0,05 à 1% en poids de cette fraction dans ledit produit.

6. Utilisation selon la revendication 5, caractérisée par le fait qu'on incorpore au produit alimentaire ou cosmétique un mélange broyé de la fraction antioxygène et de 5 à 40%, calculé sur le poids de la fraction antioxygène, d'acide citrique ou ascorbique ou leurs sels et esters.

**Patentansprüche**

1. Verfahren zur Herstellung einer oxydationsverhindernde Substanzen enthaltenden Fraktion aus einem pflanzlichen Material, das reich an diesen Substanzen ist, gekennzeichnet dadurch,

— daß man das zuvor gemahlene pflanzliche Material in einem Öl aufschlämmt,
— daß man die Mischung feinstvermahlt, so daß Teilchen mit einer Größe von höchstens 20 Mikron erhalten werden,
— daß man eine Wärmebehandlung der feinstgemahlenen Mischung bei 200—250°C während 1 bis 5 Minuten oder bei 100—200°C während 5 bis 10 Minuten durchführt und daß man den verkohlten Rückstand abtrennt, bevor oder nachdem man einen Destillationsträger, wie ein Mono-, Di- oder Triglycerid mittlerer Kettenlänge, dazu beigegeben hat und sie eventuell durch die Hinzufügung von Öl standardisiert hat, derart, daß diese 10 bis 30 Gew.-% des pflanzlichen Materials und 1 bis 20 Gew.-% des Destillationsträgers enthält, und
— daß man die wärmebehandelte Suspension einer Molekulardestillation unter einem Unterdruck von höchstens 0,67 Pa bei einer Temperatur von 190—280°C und mit einem Massendurchsatz von 0,5 bis 7 kg/h unterwirft, und daß man ein Kondensat auffängt, das die oxydationsverhindernden Prinzipien, den Destillationsträger und einen Teil des verwendeten Öles enthält.

2. Verfahren gemäß Anspruch 1, dadurch gekennzeichnet, daß das behandelte pflanzliche Material aus Rosmarin- oder Salbeiblättern oder Kakaohülsen besteht.
3. Verfahren gemäß Anspruch 1, dadurch gekennzeichnet, daß das behandelte pflanzliche Material aus Rückständen der Destillation von etherischem Öl aus Rosmarin besteht.
4. Verfahren gemäß Anspruch 1, dadurch gekennzeichnet, daß man der Suspension 0,5 bis 5 Gew.-% Wasser vor der Feinstvermahlung beimischt.
5. Verwendung der die nach einem der Ansprüche 1 bis 4 erhaltenen oxydationsverhindernden Substanzen enthaltenden Fraktion zum Schützen eines Lebensmittelproduktes oder kosmetischen Produktes vor Oxydation, dadurch gekennzeichnet, daß man 0,05 bis 1 Gew.-% dieser Fraktion dem besagten Produkt zusetzt.
6. Verwendung gemäß Anspruch 5, dadurch gekennzeichnet, daß man dem Lebensmittelprodukt oder kosmetischen Produkt eine gemahlene Mischung aus der oxydationsverhindernden Fraktion und 5 bis 40% Zitronen- oder Askorbinsäure oder ihrer Salze und Ester, bezogen auf das Gewicht der oxydationsverhindernden Fraktion, zusetzt.

**Claims**

1. A process for the production of a fraction containing oxidation-inhibiting substances from a vegetable material rich in these substances, characterised in that

— the previously ground vegetable material is suspended in an oil,
— the mixture is micronised in order to obtain particles of at most 20 microns in size,
— the micronised mixture is subjected to a heat treatment at 200—250°C during 1 to 5 min. or at 100—200°C during 5 to 30 min. and the carbonized residue is separated, before or after having added a distillation vehicle such as a mono-, di- or triglyceride of medium chain length and optionally having added oil thereto so that it contains 10 to 30% by weight of vegetable material and 1 to 20% by weight of distillation vehicle, and
— the heat treated suspension is subjected to molecular distillation under a vacuum of at most 0.67 Pa at a temperature of 190—280°C and with a throughput of material of 0.5 to 7 kg/h, and a condensate containing the oxidation-inhibiting principles, the distillation vehicle and some of the oil used is collected.

2. A process as claimed in claim 1, characterised in that the vegetable material treated is formed by leaves of rosemay or sage or by cocoa skins.
3. A process as claimed in claim 1, characterised in that the vegetable material treated is formed by residues from the distillation of the essential oil of rosemary.
4. A process as claimed in claim 1, characterised in that from 0.5 to 5% by weight of water is added to the suspension before micronisation.
5. The use of the fraction containing oxidation-inhibiting substances obtained by the process as claimed in anyone of claims 1 to 4 for preserving a food or cosmetic product from oxidation, characterised in that 0.05 to 1% by weight of said fraction is incorporated therein.

7

## 0 009 661

6. The use as claimed in claim 5, characterised in that a ground mixture of the oxidation-inhibiting fraction with 5 to 40%, based on the weight of the oxidation-inhibiting fraction, or ascorbic or citric acid or their salts and esters is incorporated therein.

8